(19) Europäisches Patentamt / European Patent Office / Office européen des brevets

(11) **EP 4 109 493 A1**

(12) **EUROPEAN PATENT APPLICATION**
published in accordance with Art. 153(4) EPC

(43) Date of publication:
**28.12.2022 Bulletin 2022/52**

(21) Application number: **21757272.6**

(22) Date of filing: **15.02.2021**

(51) International Patent Classification (IPC):
**H01J 65/00** *(2006.01)*        **A61L 2/10** *(2006.01)*
**H01J 61/16** *(2006.01)*

(52) Cooperative Patent Classification (CPC):
**A61L 2/10; H01J 61/16; H01J 65/00;** Y02A 50/30

(86) International application number:
**PCT/JP2021/005445**

(87) International publication number:
**WO 2021/166830 (26.08.2021 Gazette 2021/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.02.2020 JP 2020025620**
**03.06.2020 JP 2020096731**

(71) Applicant: **Ushio Denki Kabushiki Kaisha Tokyo 100-8150 (JP)**

(72) Inventor: **NAITO, Keisuke Tokyo 100-8150 (JP)**

(74) Representative: **Tomerius, Isabel Lang & Tomerius Patentanwaltspartnerschaft mbB Rosa-Bavarese-Strasse 5 80639 München (DE)**

(54) **BACTERIOSTATIC METHOD**

(57)    Provided is a bacteriostatic method for suppressing the proliferation of a bacterium while reducing the influence on a human body.

The bacteriostatic method is for suppressing a proliferation of a bacterium in a target region and includes a step (a) for irradiating the target region with ultraviolet light having a main peak wavelength of 200 nm to 230 nm inclusive at an average irradiance equal to or less than $D_{Max}$ ($\mu$W/cm$^2$) defined by Formula (1):

$$D_{Max} = 9391.1 \times \exp(-0.043\lambda) \cdots (1)$$

where $\lambda$ is the main peak wavelength (nm).

**Fig. 1**

**Description**

TECHNICAL FIELD

The present invention relates to a bacteriostatic method, and more particularly to a bacteriostatic method using ultraviolet light.

BACKGROUND ART

Conventionally, a technique described in Patent Document 1 below is known as a sterilization method using ultraviolet light. Patent Document 1 below indicates that irradiation of ultraviolet light of 0.008-0.17 mW/cm$^2$ (8-170 $\mu$W/cm$^2$) can kill Escherichia coli and molds.

PRIOR ART DOCUMENT

PATENT DOCUMENT

Patent Document 1: JP-A-2014-136113

SUMMARY OF THE INVENTION

PROBLEMS TO BE SOLVED BY THE INVENTION

It is known that DNA exhibits the highest absorption characteristics around a wavelength of 260 nm. A low-pressure mercury lamp exhibits a high emission spectrum around a wavelength of 254 nm. Therefore, when sterilization treatment using ultraviolet light is performed, a low-pressure mercury lamp is commonly used as a light source.
Although the above Patent Document 1 does not describe the wavelengths in a strict sense, in light of the fact that it is related to sterilization technology, the fact that it simply specifies the name "ultraviolet lamp" without any special explanation, and the content of the experimental results, it is assumed that a low-pressure mercury lamp is used as the light source in Patent Document 1.
However, when irradiated with light around a wavelength of 254 nm, a human body may be adversely affected. That is, the sterilization method described in Patent Document 1 can be used only when a human is surely not present in a region to be sterilized. Therefore, in order to prevent an accident by any chance, the sterilization method is limited to be used in a specific environment in which a human having specialized knowledge is present.
Given the above problems, an object of the present invention is to provide a bacteriostatic method for suppressing the proliferation of bacteria while suppressing an influence on a human body.

MEANS FOR SOLVING THE PROBLEMS

There is a demand for suppressing the proliferation of bacteria in places where bacteria such as molds are likely to grow, such as a wet area in the home including a bathroom, a toilet, a washroom, and a kitchen, and a filter of an air conditioner. In addition, in hospitals and the like, there is also a demand for suppressing the proliferation of bacteria from the viewpoint of enhancing the ability to prevent nosocomial infection.
Here, when the proliferation of bacteria is suppressed, it is certainly preferable that existing bacteria can be completely eliminated, that is, sterilized. However, even if the "sterilization" state cannot be achieved, there are great advantages such as ease of cleaning and reduction in the frequency of cleaning if a so-called "bacteriostatic" state in which the proliferation of bacteria is suppressed can be achieved.
The present invention provides a bacteriostatic method for suppressing a proliferation of a bacterium in a target region, the method including a step (a) for irradiating the target region with ultraviolet light having a main peak wavelength of 200 nm to 230 nm inclusive at an average irradiance equal to or less than $D_{Max}$ ($\mu$W/cm$^2$) defined by Formula (1):

$$D_{Max} = 9391.1 \times \exp{(-0.043\lambda)} \bullet \bullet \bullet (1)$$

where $\lambda$ is the main peak wavelength (nm).
If radiated to a human skin, ultraviolet light in a wavelength band having a main peak wavelength of 200 nm to 230 nm inclusive is absorbed by the stratum corneum of the skin and does not advance further inside (toward the basal layer). The corneocyte contained in the stratum corneum is dead as a cell. Therefore, there is almost no risk that, as in the case of irradiation with ultraviolet light having a wavelength of 254 nm, for example, ultraviolet light is absorbed into living cells such as the stratum spinosum, the stratum granulosum, and the dermis to destroy DNA.

According to the above method, the bacteriostatic effect is achieved by radiating ultraviolet light having a wavelength that has a smaller impact on the human body than the wavelength band of ultraviolet light emitted from a low-pressure mercury lamp with an irradiance extremely smaller than the irradiance of light emitted by the conventional sterilization method. As a result, the proliferation of bacteria in the target region is suppressed with the influence on a human body being reduced as much as possible. This point will be described later in the section of "MODE FOR CARRYING OUT THE INVENTION".

In the present specification, the term "bacterium" indicates a concept including a microbe and a mold (fungus).

As a light source of the ultraviolet light, an excimer lamp in which a light-emitting gas containing Kr and Cl is sealed. In that case, the main peak wavelength of the ultraviolet light from the excimer lamp is around 222 nm.

The step (a) may include irradiating the target region with the ultraviolet light at an irradiance equal to or less than 1 $\mu$W/cm$^2$.

The bacterium may be Staphylococcus aureus, and the step (a) may include irradiating the target region with the ultraviolet light at an irradiance equal to or greater than 0.4 $\mu$W/cm$^2$.

The bacterium may be a mold, and the step (a) may include irradiating the target region with the ultraviolet light at an irradiance equal to or greater than 0.29 $\mu$W/cm$^2$.

The step (a) may be intermittently performed at an interval of two hours or less.

As will be described later in the section of "MODE FOR CARRYING OUT THE INVENTION", it has been confirmed that the bacteriostatic effect is reduced when two hours or more elapse after the last execution of the step (a) for radiating ultraviolet light. According to the above method, a high bacteriostatic effect can be achieved for the target region without constantly irradiating the target region with ultraviolet light.

The bacteriostatic method may include:

a step (b) for detecting whether or not a human is present in the target region by a human sensor; and
a step (c) for irradiating the target region with the ultraviolet light at an average irradiance higher than $D_{Max}$ ($\mu$W/cm$^2$) defined by the Formula (1), wherein,
when it is detected in the step (b) that a human is present in the target region, the method may transition from the step (c) to the step (a).

According to the method described above, in a case where a human is not present in the target region, the ultraviolet light is radiated at an irradiance higher than $D_{Max}$ ($\mu$W/cm$^2$) that may affect the human body, and in a case where a human is present in the target region, the irradiance of the ultraviolet light is reduced to $D_{Max}$ ($\mu$W/cm$^2$) or less. This further enhances the effect of suppressing the proliferation of bacteria in the target region while suppressing the influence on the human body.

EFFECT OF THE INVENTION

According to the present invention, it is possible to suppress the proliferation of bacteria while suppressing the influence on the human body.

BRIEF DESCRIPTION OF THE DRAWINGS

Fig. 1 is a diagram schematically illustrating an implementation state of a bacteriostatic method according to the present invention.
Fig. 2 is a perspective view schematically illustrating an example of an external appearance of an ultraviolet light irradiation device.
Fig. 3 is an exploded perspective view of the ultraviolet light irradiation device in Fig. 2 in which a main body casing part and a lid part of a lamp house are removed from each other.
Fig. 4 is a plan view schematically illustrating a positional relationship between an excimer lamp and electrode blocks.
Fig. 5 is a diagram illustrating a spectrum of ultraviolet light emitted from the excimer lamp in which a light-emitting gas contains KrCl.
Fig. 6 is a graph for describing a threshold irradiance $D_{Max}$ ($\mu$W/cm$^2$) defined by Formula (1) described below.
Fig. 7 is another diagram schematically illustrating an implementation state of the bacteriostatic method according to the present invention.

Fig. 8A illustrates photographs showing results of Comparative Example 1 in Verification 1.

Fig. 8B illustrates photographs showing results of Comparative Example 2 in Verification 1.

Fig. 8C illustrates photographs showing results of Example 1 in Verification 1.

Fig. 9A illustrates photographs showing results of Comparative Example 3 (spore liquid of Cladosporium) in Verification 2.

Fig. 9B illustrates photographs showing results of Comparative Example 3 (strain of Cladosporium) in Verification 2.

Fig. 9C illustrates photographs showing results of Example 2 (spore liquid of Cladosporium) in Verification 2.

Fig. 9D illustrates photographs showing results of Example 2 (strain of Cladosporium) in Verification 2.

Fig. 9E illustrates photographs showing results of Comparative Example 4 (spore liquid of Cladosporium) in Verification 2.

Fig. 9F illustrates photographs showing results of Comparative Example 4 (strain of Cladosporium) in Verification 2.

Fig. 9G illustrates photographs showing results of Example 3 (spore liquid of Cladosporium) in Verification 2.

Fig. 9H illustrates photographs showing results of Example 3 (strain of Cladosporium) in Verification 2.

Fig. 9I illustrates photographs showing results of spore liquids of Cladosporium in a contaminated state (#2) in Example 4, Example 6, and Example 7.

Fig. 9J illustrates photographs showing results of strains of Cladosporium in a contaminated state (#2) in Example 4, Example 6, and Example 7.

## MODE FOR CARRYING OUT THE INVENTION

An embodiment of the bacteriostatic method according to the present invention will be described with reference to the drawings as appropriate.

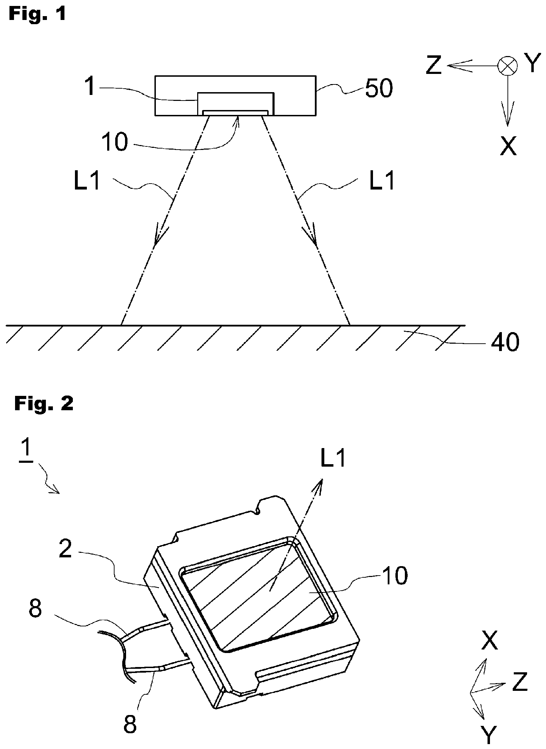

Fig. 1 is a diagram schematically illustrating an implementation state of the bacteriostatic method according to the present invention. The bacteriostatic method according to the present invention relates to a method for suppressing the proliferation of bacteria in a target region 40 by irradiating the target region 40 with ultraviolet light L1 from an ultraviolet light irradiation device 1. Fig. 1 schematically illustrates a state in which the ultraviolet light irradiation device 1 is mounted on a housing 50, and the target region 40 is irradiated with the ultraviolet light L1 from a light extraction surface 10 of the ultraviolet light irradiation device 1.

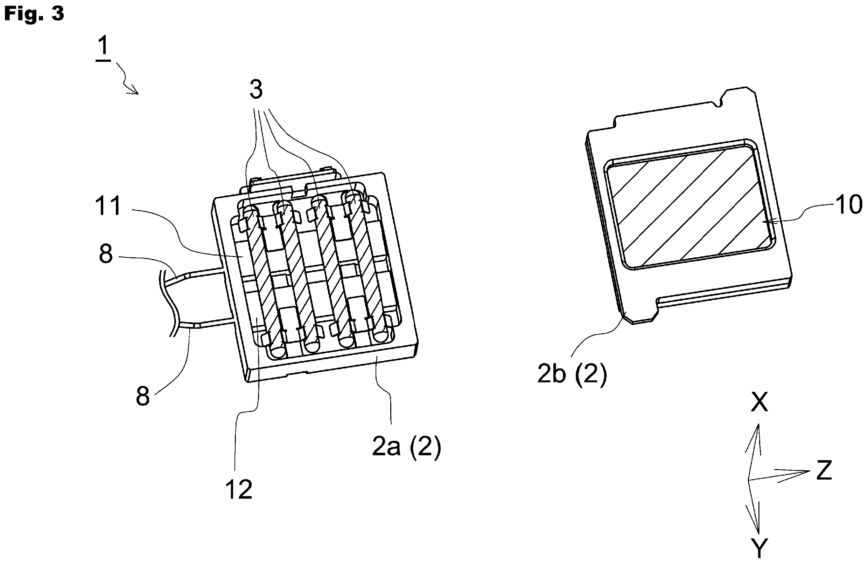

Fig. 2 is a perspective view schematically illustrating the external appearance of the ultraviolet light irradiation device 1. Fig. 3 is an exploded perspective view of the ultraviolet light irradiation device 1 in Fig. 2 in which a main body casing part 2a and a lid part 2b of a lamp house 2 are removed from each other. However, the structure of the ultraviolet light irradiation device 1 described below is merely an example, and the structure of a light source used for the bacteriostatic method according to the present invention is not limited.

Each of the following drawings will be described with reference to an X-Y-Z coordinate system in which a direction of extracting the ultraviolet light L1 is defined as an X direction, and a plane orthogonal to the X direction is defined as a YZ plane. More specifically, as will be described later with reference to Fig. 3 and subsequent drawings, the tube axis direction of an excimer lamp 3 is defined as a Y direction, and a direction orthogonal to the X direction and the Y direction is defined as a Z direction.

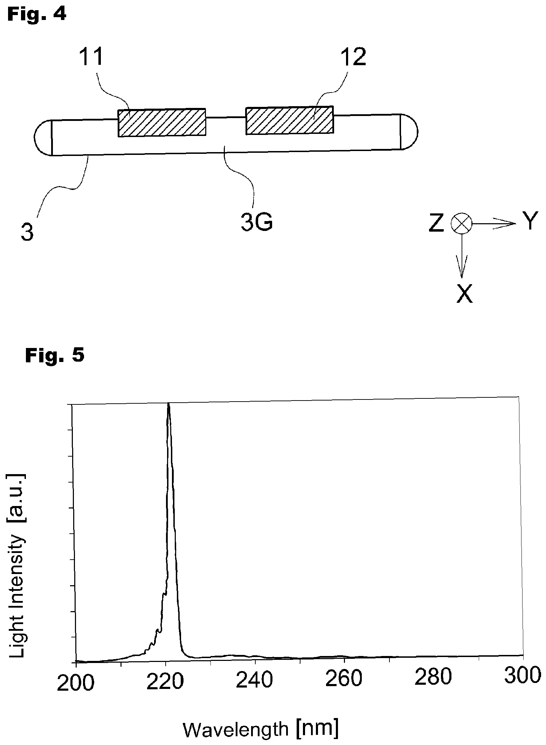

As illustrated in Figs. 2 and 3, the ultraviolet light irradiation device 1 includes the lamp house 2 which has the light extraction surface 10 formed on one surface. The lamp house 2 includes the main body casing part 2a and the lid part 2b, and the excimer lamp 3 and electrode blocks (11, 12) are accommodated in the main body casing part 2a. Fig. 3 illustrates an example where four excimer lamps 3 are accommodated in the lamp house 2. The electrode blocks (11, 12) are electrically connected to a feeder 8 and constitute an electrode for supplying power to each excimer lamp 3. Fig. 4 is a plan view schematically illustrating a positional relationship between the excimer lamps 3 and the electrode blocks (11, 12).

As illustrated in Figs. 2 to 4, the ultraviolet light irradiation device 1 according to the present embodiment includes two electrode blocks (11, 12) which are arranged so as to be in contact with the outer surface of a light-emitting tube of each excimer lamp 3. The electrode blocks (11, 12) are disposed of at positions separated from each other in the Y direction. The electrode blocks (11, 12) are made of a conductive material, preferably a material exhibiting reflectivity to ultraviolet light emitted from the excimer lamps 3. As an example, the electrode blocks (11, 12) are both made of Al, an Al alloy, stainless steel, or the like. The electrode blocks (11, 12) are disposed of to extend across the excimer lamps 3 in the Z direction while being in contact with the outer surfaces of the light-emitting tubes of the excimer lamps 3.

Each of the excimer lamps 3 has a light-emitting tube with the tube axis direction extending along the Y direction, and the outer surfaces of the light-emitting tubes of the excimer lamps 3 are in contact with the electrode blocks (11, 12) at positions separated in the Y direction. A light-emitting gas 3G is sealed in the light-emitting tube of each excimer lamp 3. When a high-frequency AC voltage of, for example, about 10 kHz to 5 MHz is applied between the electrode blocks (11, 12) through the feeder 8 (see Fig. 2), the voltage is applied to the light-emitting gases 3G via the light-emitting tubes of the excimer lamps 3. At this time, discharge plasma is generated in a discharge space in which the light-emitting gas 3G is sealed, so that atoms of the light-emitting gas 3G are excited to be brought into an excimer state, and excimer light emission occurs when the atoms shift to the ground state.

The light-emitting gas 3G is made of a material that emits the ultraviolet light L1 having a main emission wavelength of 200 nm to 230 nm inclusive at the time of excimer emission. As an example, the light-emitting gas 3G includes KrCl or KrBr. In addition to the above-mentioned gas species, an inert gas such as argon (Ar) or neon (Ne) may be mixed.

For example, when the light-emitting gas 3G contains KrCl, the excimer lamp 3 emits the ultraviolet light L1 having a main peak wavelength of around 222 nm. When the light-emitting gas 3G contains KrBr, the excimer lamp 3 emits the ultraviolet light L1 having a main peak wavelength of around 207 nm. Fig. 5 is a diagram illustrating a spectrum of the ultraviolet light L1 emitted from the excimer lamp 3 in which the light-emitting gas 3G contains KrCl.

When the bacteriostatic method according to the present invention is performed, a step (a) for irradiating the target region 40 with the ultraviolet light L1 from the ultraviolet light irradiation device 1 at an average irradiance of less than or equal to a threshold irradiance $D_{Max}$ ($\mu$W/cm$^2$) defined by following Formula (1) is performed. In Formula (1), $\lambda$ is the main peak wavelength (nm) of the ultraviolet light L1. That is, $\lambda$ = 222 nm when the light source mounted on the ultraviolet light irradiation device 1 is the excimer lamp 3 in which the light-emitting gas 3G contains KrCl.

$$D_{Max} = 9391.1 \times \exp(-0.043\lambda) \bullet \bullet \bullet (1)$$

Note that exp (x) is synonymous with e$^x$.

The ultraviolet light irradiation device 1 may include a control unit (not illustrated), and this control unit may perform energization control for each electrode block (11, 12) through the feeder 8. The control unit controls power supplied to each electrode block (11, 12) so that the average irradiance of the ultraviolet light L1 for the target region 40 becomes equal to or less than $D_{Max}$ ($\mu$W/cm$^2$).

When the target region 40 is continuously irradiated with the ultraviolet light L1 from the ultraviolet light irradiation device 1, the irradiance may be set to be equal to or less than $D_{Max}$ ($\mu$W/cm$^2$). On the other hand, as will be described later, when the ultraviolet light irradiation device 1 is intermittently turned on, the average irradiance for the target region 40 is set to be equal to or less than $D_{Max}$ ($\mu$W/cm$^2$).

As described above in the section of "PROBLEMS TO BE SOLVED BY THE INVENTION", it is known that ultraviolet light around 260 nm exhibiting high absorption characteristics in DNA has a great influence on the human body. For this reason, regulations are imposed on the handling of ultraviolet light, and it is currently recommended to set ultraviolet lightto be within the regulation value defined by the American Conference of Governmental Industrial Hygienists (ACGIH). Table 1 below shows threshold limit values (TLVs) defined by ACGIH. The TLV indicates an upper limit value at which exposure within an eight-hour workday is allowed, considering that industrial workers commonly work eight hours a day.

[Table 1]

| Wavelength (nm) | TLV (mJ/cm$^2$) |
| --- | --- |
| 200 | 100 |
| 210 | 40 |
| 220 | 25 |
| 230 | 16 |
| 240 | 10 |
| 250 | 7 |
| 254 | 6 |
| 260 | 4.6 |
| 270 | 3 |

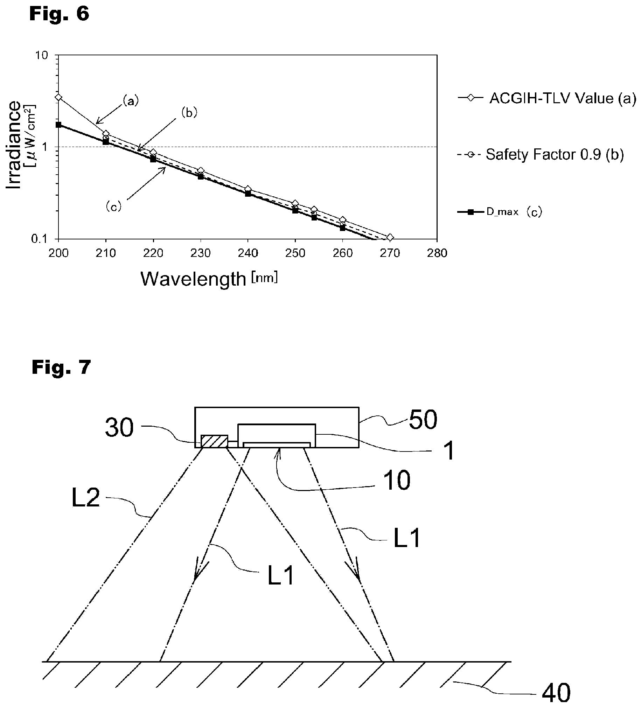

The values shown in Table 1 are specified radiation exposure doses for eight hours. A graph obtained by converting these values per unit time (second) corresponds to the plot line indicated by (a) in Fig. 6. As shown in Table 1, the ACGIH discretely specifies the radiation exposure dose for each wavelength. Therefore, the graph of Fig. 6(a) is illustrated by converting the value for each wavelength into a value per unit time (second) and performing linear interpolation.

In Fig. 6, the graph of (b) is created based on a value obtained by multiplying the ACGIH-TLV value shown in (a) by a safety factor of 0.9. On the other hand, the graph of (c) is a curve defined by the above Formula (1). As illustrated in Fig. 6, it can be seen that the graph (c) does not exceed the value of the graph (b) based on the value obtained by multiplying the ACGIH-TLV value by the safety factor of 0.9 within the range of 200 nm to 270 nm.

That is, even if a human is present in the target region 40, the influence on the human body can be suppressed by irradiating the target region 40 with the ultraviolet light L1 from the ultraviolet light irradiation device 1 at an average irradiance equal to or less than $D_{Max}$ ($\mu$W/cm$^2$) defined by above Formula (1). In particular, in a case where the target region 40 is a place where the same person is less likely to stay for more than eight hours, the influence on the body of a person who is present in the target region 40 is extremely low.

For example, if the target region 40 is a wet area in the home such as a bathroom, a toilet, a washroom, or a kitchen, or a filter of an air conditioner, it is exceedingly unlikely that the same person stays in such a place for more than eight hours. When the method of the present invention is used for a bacteriostatic treatment from the viewpoint of prevention of nosocomial infection in a hospital, the method can also ensure the bacteriostatic effect while suppressing the influence on the human body by irradiating a region where the same person is unlikely to stay for more than eight hours with the ultraviolet light L1 at an irradiance equal to or less than $D_{Max}$ ($\mu$W/cm$^2$).

It is a newly discovered finding through intensive research by the present inventor that when the ultraviolet light L1 has a main peak wavelength of 200 nm to 230 nm inclusive, the effect of suppressing the proliferation of bacteria in the target region 40 can be obtained even with an extremely low irradiance such as an irradiance equal to or less than $D_{Max}$ ($\mu$W/cm$^2$) defined by above Formula (1). According to Fig. 6, this value is lower than 1 $\mu$W/cm$^2$, in the case where, for example, the ultraviolet light L1 having a main peak wavelength of 222 nm is used. The effect of suppressing the proliferation of bacteria by such extremely weak ultraviolet light L1 has not been previously known and is a quite surprising effect. This respect will be described later with reference to Examples.

In addition, since the ultraviolet light L1 having a main peak wavelength of 200 nm to 230 nm inclusive is hardly absorbed by oxygen, ozone is not generated, and from this point, it can be said that the ultraviolet light L1 has less effect on the human body.

As illustrated in Fig. 7, the housing 50 may include a human sensor 30 capable of detecting the presence of a human in the target region 40 in addition to the ultraviolet light irradiation device 1. When detecting that a human is present in the target region 40 (step (b)), the human sensor 30 outputs a signal indicating the presence of the human in the target region 40 to the control unit included in the ultraviolet light irradiation device 1, and the control unit performs control to reduce the irradiance of the ultraviolet light L1 emitted from the ultraviolet light irradiation device 1 (step (c)).

That is, in the mode illustrated in Fig. 7, when a human is not present in the target region 40, the ultraviolet light irradiation device 1 irradiates the target region 40 with the ultraviolet light L1 at an irradiance exceeding $D_{Max}$ ($\mu$W/cm$^2$) defined by above Formula (1). Thus, an intense bacteriostatic effect and sterilizing effect in the target region 40 are achieved. On the other hand, when the human sensor 30 detects that a human is present (can be present) in the target region 40, the irradiance of the ultraviolet light L1 from the ultraviolet light irradiation device 1 in the target region 40 is reduced to $D_{Max}$ ($\mu$W/cm$^2$) defined by above Formula (1) or less. Thus, even when a human is present in the target region 40, the bacteriostatic effect in the target region 40 is ensured.

Bacteria exhibit the property of growing over time unless they are completely killed. In addition, when an object is left untreated after being sterilized once, bacteria likely adhere from the outside over time. Then, once the bacteria grow and form colonies, it takes a lot of work to remove the bacteria. However, due to the configuration in which the target region 40 is irradiated with the ultraviolet light L1 at an irradiance with a level having less effect on the human body as in the above method, the proliferation of bacteria is suppressed, though the bacteria cannot be completely killed. Therefore, the work of removing the bacteria can be greatly reduced. In particular, by continuing irradiation with the ultraviolet light L1 at an irradiance equal to or less than $D_{Max}$ ($\mu$W/cm$^2$) defined by above Formula (1) after performing the sterilization treatment, it is possible to prevent the proliferation of bacteria in the target region 40 while suppressing the influence on the human body.

From the viewpoint of ensuring higher safety, control to emit the ultraviolet light L1 only when it is confirmed by the human sensor 30 that no human is present in the target region 40 may be performed. Specifically, the ultraviolet light irradiation device 1 may irradiate the target region 40 with the ultraviolet light L1 at an irradiance equal to or lower than $D_{Max}$ ($\mu$W/cm$^2$) defined by the above Formula (1) only when no human is present in the target region 40, and may stop the irradiation with the ultraviolet light L1 when it is confirmed by the human sensor 30 that a human is present in the target region 40.

[Verification]

The effect that the proliferation of bacteria in the target region 40 can be suppressed with the ultraviolet light L1 having an extremely low irradiance equal to or less than $D_{Max}$ ($\mu$W/cm$^2$) defined by above Formula (1) for the target region 40 will be described below with reference to Examples.

(Verification 1: Staphylococcus aureus)

An experiment was conducted using Staphylococcus aureus. In order to simulate a habitat environment of bacteria, the experiment was conducted in three patterns: a no-load state (ideal state), a clean state, and a contaminated state. The EN test method which is a basic test method for evaluating bactericidal activity specifies a clean state and a contaminated state as states indicating different contamination levels assumed in a target region. These states correspond to the state in which a load substance is added to simulate the contaminated state. In Verification 1, a no-load state (ideal state) having no load substance was also examined in addition to these two states.

As each of the samples of the no-load state, the clean state, and the contaminated state, a sample prepared by adding, as a model stain, a protein (bovine serum albumin (BSA)) to a Staphylococcus aureus solution and smearing the solution on a standard agar medium in a petri dish was used. Table 2 below shows the amounts of Staphylococcus aureus and BSA used for preparing these samples.

[Table 2]

| State | Staphylococcus aureus (CFU/mL) | BSA (g/L) |
|---|---|---|
| No-load state | $10^4$ | - |
| Clean state | $10^4$ | 0.6 |
| Contaminated state | $10^4$ | 6 |

In Table 2, CFU means a colony-forming unit.

The samples having the states in Table 2 were not irradiated with ultraviolet light (Comparative Example 1), were irradiated with ultraviolet light having a wavelength of 254 nm (Comparative Example 2) and were irradiated with ultraviolet light having a wavelength of 222 nm (Example 1). The states of the respective samples in Comparative Example 1, Comparative Example 2, and Example 1 were checked.

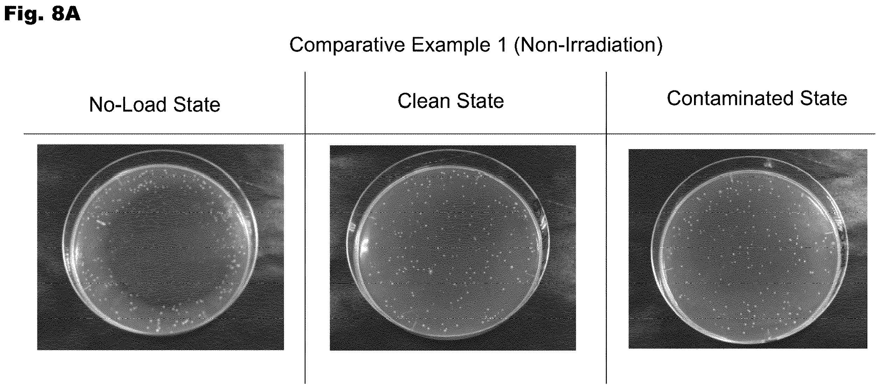

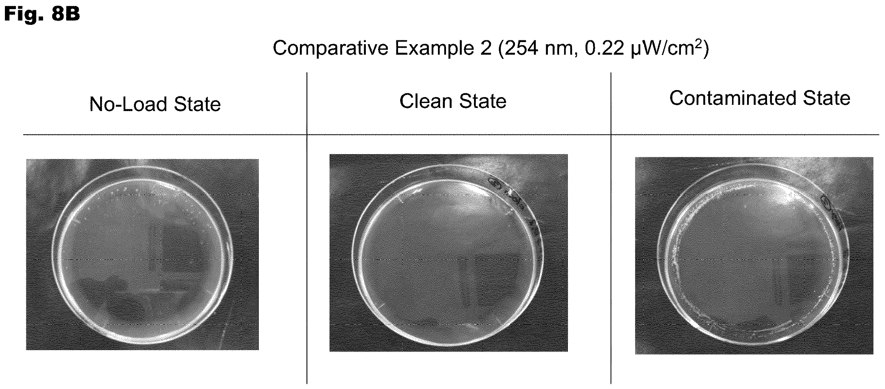

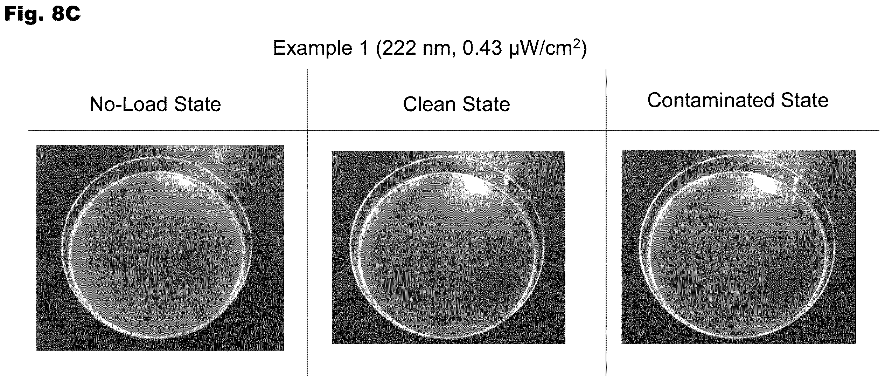

In Comparative Example 1, the samples were left untreated without doing anything. In Comparative Example 2, ultraviolet light having a wavelength of 254 nm was emitted from a low-pressure mercury lamp for 72 hours at an irradiance of 0.22 $\mu$W/cm$^2$ which was a value equal to or lower than $D_{Max}$ ($\mu$W/cm$^2$) defined by the above Formula (1). In Example 1, ultraviolet light having a wavelength of 222 nm was emitted from a KrCl excimer lamp for 72 hours at an irradiance of 0.43 $\mu$W/cm$^2$ which was a value equal to or less than $D_{Max}$ ($\mu$W/cm$^2$) defined by the above Formula (1). In Comparative Example 2 and Example 1, the samples were left overnight after the irradiation treatment, and then, checked. The results are shown in Figs. 8A to 8C and Table 3.

In the evaluation in Table 3, a sample in which the formation of colonies could not be visually observed (a sample in which the bacteriostatic effect was extremely high) is evaluated as "A", a sample in which the formation of a small amount of colonies could be observed (a sample in which the bacteriostatic effect was recognized) is evaluated as "B", and a sample in which the formation of many colonies were observed (a sample in which the proliferation of bacteria could not be suppressed) is evaluated as "C".

[Table 3]

| | | Staphylococcus aureus | | |
|---|---|---|---|---|
| | | No-load state | Clean state | Contaminated state |
| Comparative Example 1 | Not irradiated | C | C | C |
| Comparative Example 2 | 254 nm 0.22 $\mu$W/cm$^2$ | C | B | C |
| Example 1 | 222 nm 0.43 $\mu$W/cm$^2$ | A | A | A |

According to Example 1, it was confirmed that the proliferation of Staphylococcus aureus could be suppressed by continuous irradiation even with ultraviolet light having an extremely low irradiance of 0.43 $\mu$W/cm$^2$ regardless of the contaminated state. On the other hand, in both Comparative Example 1 and Comparative Example 2, it was confirmed that colonies were formed. In Comparative Example 2, the growth of colonies could be somewhat suppressed in the "clean state" in which the degree of contamination was low, but the growth of colonies could not be suppressed in the "contaminated state" in which the degree of contamination was high. In the case where the ultraviolet light of 254 nm corresponding to the main peak wavelength of ultraviolet light from a low-pressure mercury lamp commonly used as a germicidal lamp was used as in Comparative Example 2, the value of $D_{Max}$ defined by above Formula (1) was smaller than 222 nm, and thus it is considered that the bacteriostatic effect could not be sufficiently obtained despite the continuous irradiation of the ultraviolet light.

(Verification 2: Mold)

An experiment was conducted using Cladosporium (Cladosporium cladosporioides (NBRC. 6368)), Penicillium (Penicillium citrinum (NBRC 6352)), and Aspergillus niger (NBRC 105649), those of which are one of black molds. In order to simulate a habitat environment of bacteria, the experiment was conducted in three patterns: a no-load state (ideal state), a clean state, and a contaminated state, as in Verification 1.

Furthermore, in Verification 2, in order to simulate both a state in which an ungrown mold is present and a state in which the mold has already grown, two types of samples were prepared: a sample obtained by introducing a spore liquid of the mold into a culture medium; and a sample obtained by introducing physiological saline, into which a strain has been put, into a culture medium. The former simulates a case where the presence of mold is invisible but the mold is actually present, and the latter simulates a case where the mold has grown to an extent that the mold can be visually recognized.

The spore liquid was adjusted from $10^4$/mL to $10^6$/mL in accordance with the test for fungus resistance defined by JIS Z 2911. As a medium, a commercially available potato dextrose agar medium (PDA medium) was used and introduced into the petri dish. In addition, for each sample in a clean state and a contaminated state, a sample obtained by smearing, as a model stain, BSA onto an agar medium was used. Table 4 below summarizes the conditions for creating the samples prepared in Verification 2.

[Table 4]

| No. | | BSA (g/L) | Use of strain | Load state |
|---|---|---|---|---|
| #1 | Spore liquid 50 $\mu$L | 0.6 | - | Clean |
| #2 | Spore liquid 50 $\mu$L | 6 | - | Contaminated |
| #3 | Physiological saline 50 $\mu$L | 0.6 | Strain | Clean |
| #4 | Physiological saline 50 $\mu$L | 6 | Strain | Contaminated |
| #5 | - | - | Strain | No-load |
| #6 | Spore liquid 50 $\mu$L + physiological saline 50 $\mu$L | - | - | No-load |

In Table 4 above, the samples #1 to #6 in each state were checked when they were not irradiated with UV light (Comparative Examples 3 to 4) and when they were irradiated with UV light at a wavelength of 222 nm (Examples 2 to 8). Note that the season in which the verification of Comparative Example 3 and Example 2 was performed and the season in which the verification of Comparative Example 4 and Examples 3 to 8 was performed are different from each other, and thus the conditions of the temperature and humidity of the atmosphere at the time of verification are different. Specifically, during the verification of Comparative Example 3 and Example 2, the ambient temperature was 15 to 18°C, and the humidity was about 30% RH. In addition, during the verification of Comparative Example 4 and Example 3 to 8, the ambient temperature was 22 to 26°C, and the humidity was about 40% RH.

The irradiation conditions of Examples 2 to 8 will be described below. Examples 2 and 3 correspond to continuous irradiation, and Examples 4 to 8 correspond to intermittent irradiation.

[Continuous irradiation]

(Example 2)

The samples were irradiated with ultraviolet light having a wavelength of 222 nm from a KrCl excimer lamp for 72 hours at an irradiance of 0.76 $\mu$W/cm$^2$ which was a value equal to or less than $D_{Max}$ ($\mu$W/cm$^2$) defined by the above Formula (1).

(Example 3)

The samples were irradiated with ultraviolet light under the same conditions as Example 2 except that the irradiance was set to 0.29 $\mu$W/cm$^2$.

[Intermittent irradiation]

(Example 4)

Example 4 was conducted under the same conditions as Example 2, except that an operation for irradiating the samples with ultraviolet light at an irradiance of 0.76 $\mu$W/cm$^2$ for 45 minutes and then stopping the irradiation for 15 minutes was repeated for 72 hours. In this case, the average irradiance during 72 hours corresponds to 0.57 $\mu$W/cm$^2$.

(Example 5)

The cycle of the irradiation time was set to be different from that in Example 4. Specifically, Example 5 was conducted under the same conditions as Example 4, except that an operation for irradiating the samples for 30 minutes and then stopping the irradiation for 30 minutes was repeated. In this case, the average irradiance during 72 hours corresponds to 0.38 $\mu$W/cm$^2$.

(Example 6)

The cycle of the irradiation time was set to be different from that in Example 4. Specifically, Example 6 was conducted under the same conditions as Example 4, except that an operation for irradiating the samples for two hours and then stopping the irradiation for two hours was repeated. In this case, the average irradiance during 72 hours corresponds to 0.38 $\mu$W/cm$^2$.

(Example 7)

The cycle of the irradiation time was set to be different from that in Example 4. Specifically, Example 7 was conducted under the same conditions as Example 4, except that an operation for irradiating the samples for three hours and then stopping the irradiation for three hours was repeated. In this case, the average irradiance during 72 hours corresponds to 0.38 $\mu$W/cm$^2$.

(Example 8)

The cycle of the irradiation time was set to be different from that in Example 4. Specifically, Example 8 was conducted under the same conditions as Example 4, except that an operation for irradiating the samples for four hours and then stopping the irradiation for four hours was repeated. In this case, the average irradiance during 72 hours corresponds to 0.38 $\mu$W/cm$^2$.

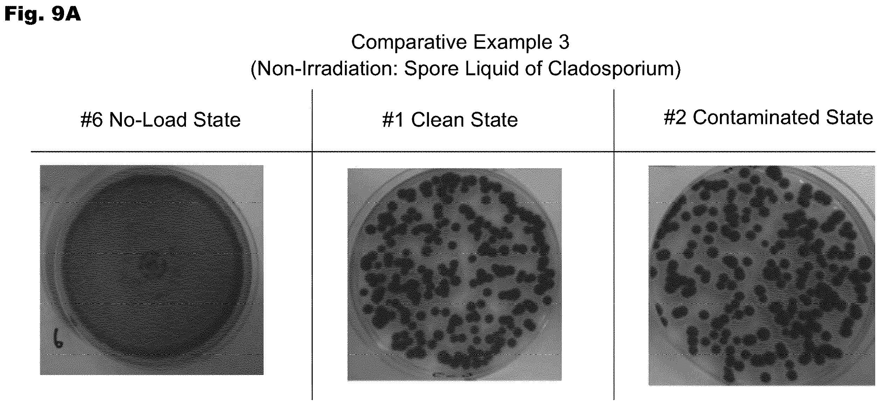

The results are shown in Tables 5 and 6 and Figs. 9A to 9H. In Table 5, the verification of the no-load state (#6) in Examples 3 to 8 is omitted. This is because it is predicted that results at least the same as or higher than the results of the clean state (#1) are obtained in the light of the results of Comparative Example 3 and Example 2.

The photographs in the drawings correspond to the following states. Since it is extremely enormous to provide all the photographs of the places showing the results indicated in Tables 5 and 6, only representative photographs of Cladosporium are extracted and shown in the drawings.

Fig. 9A illustrates photographs showing the results of Comparative Example 3 (spore liquid of Cladosporium).

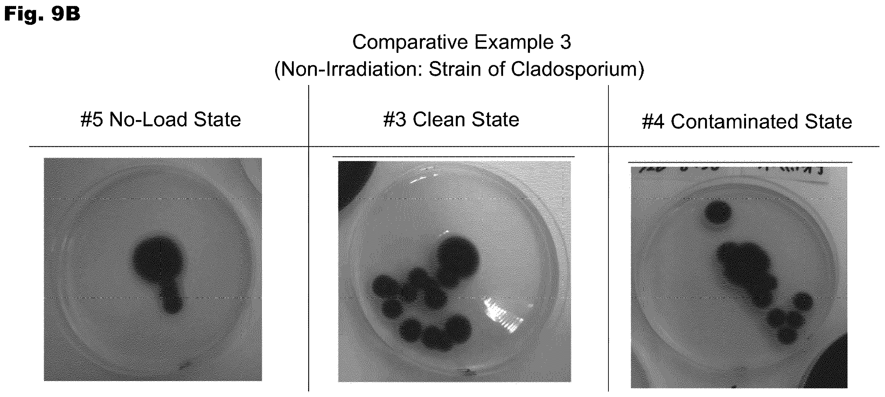

Fig. 9B illustrates photographs showing the results of Comparative Example 3 (strain of Cladosporium).

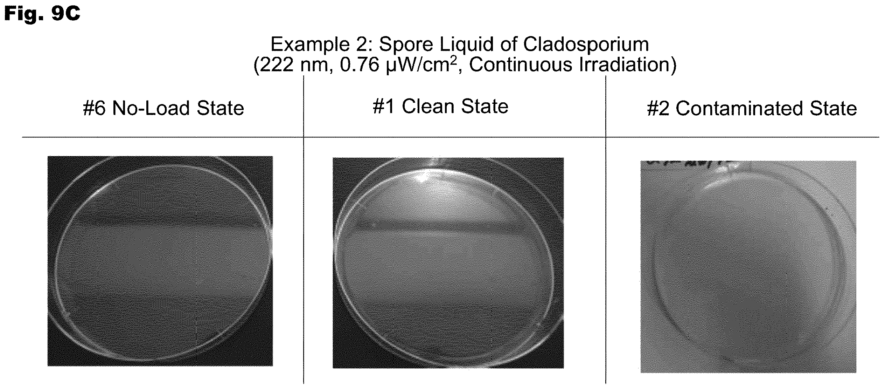

Fig. 9C illustrates photographs showing the results of Example 2 (spore liquid of Cladosporium).

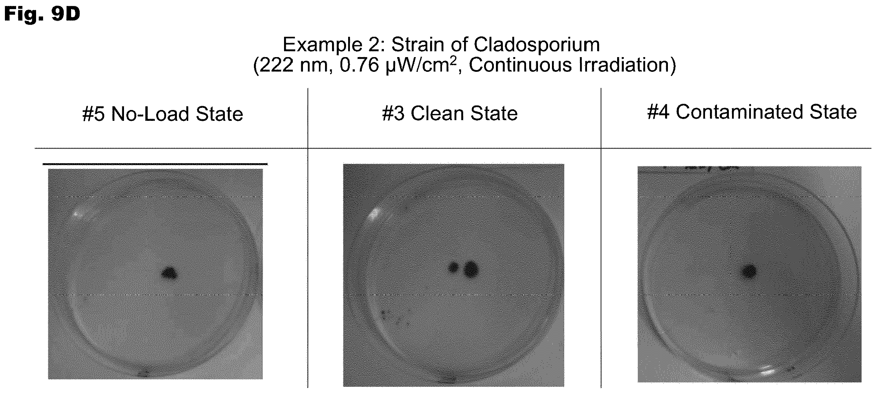

Fig. 9D illustrates photographs showing the results of Example 2 (strain of Cladosporium).

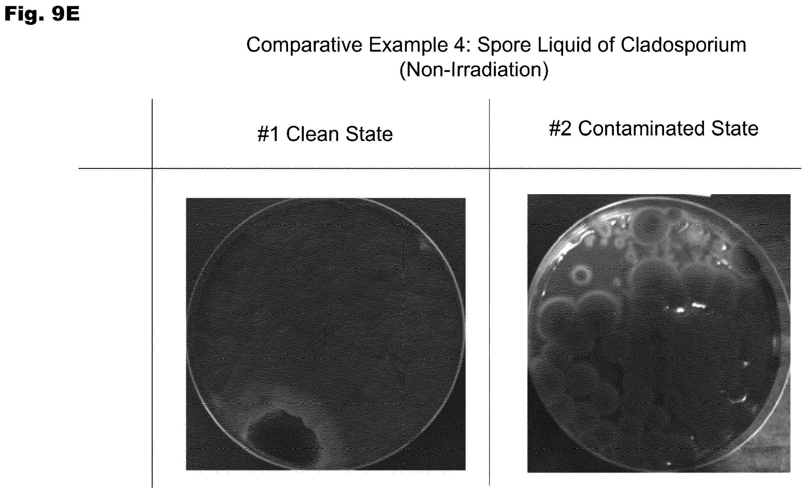

Fig. 9E illustrates photographs showing the results of Comparative Example 4 (spore liquid of Cladosporium).

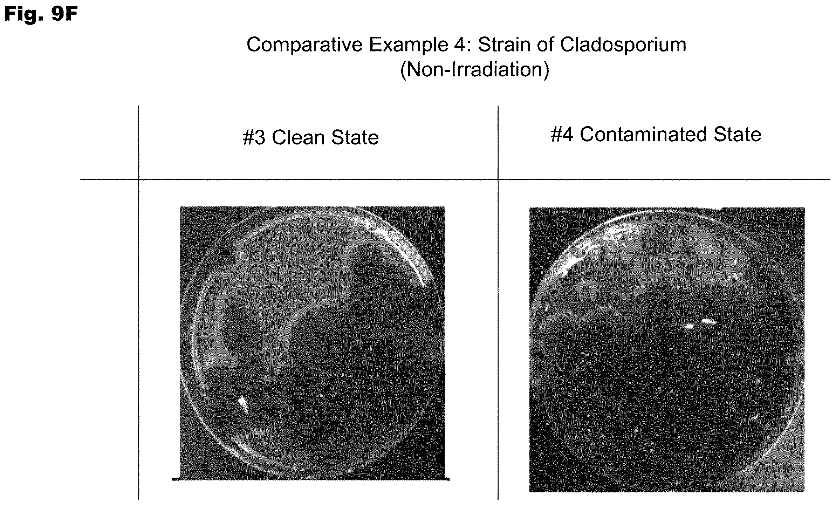

Fig. 9F illustrates photographs showing the results of Comparative Example 4 (strain of Cladosporium).

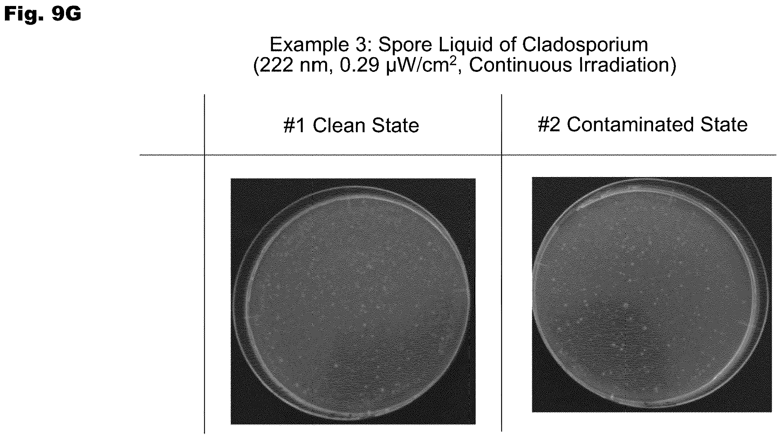

Fig. 9G illustrates photographs showing the results of Example 3 (spore liquid of Cladosporium).

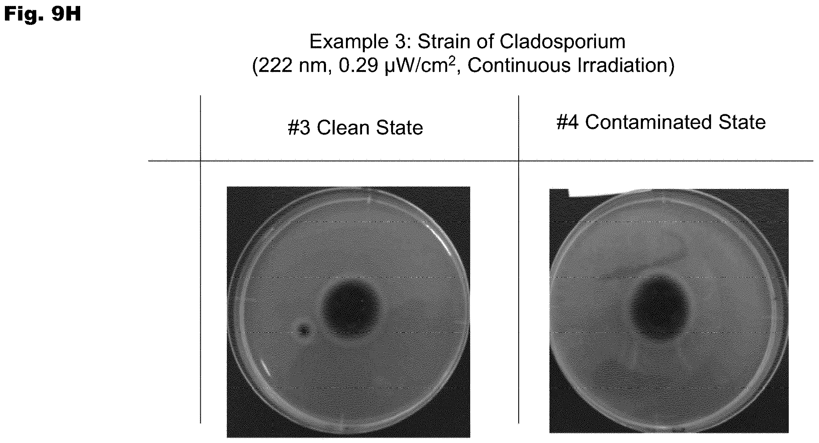

Fig. 9H illustrates photographs showing the results of Example 3 (strain of Cladosporium).

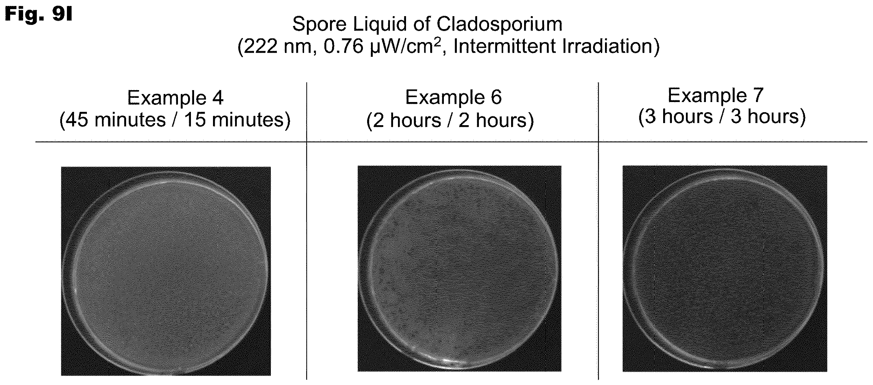

Fig. 9I illustrates photographs showing results of spore liquids of Cladosporium in a contaminated state (#2) in Example 4, Example 6, and Example 7.

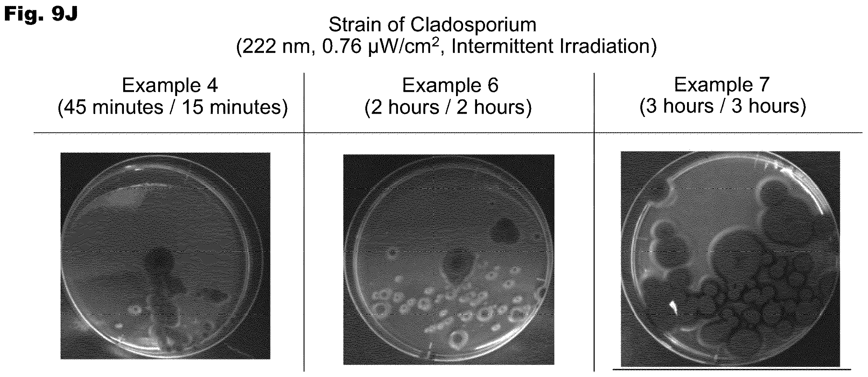

Fig. 9J illustrates photographs showing results of strain of Cladosporium in a contaminated state (#2) in Example 4, Example 6, and Example 7.

The expression "45 min/15 min" in Figs. 9I and 9J mean that a cycle of emitting ultraviolet light for 45 minutes and then stopping the irradiation for 15 minutes is repeated. The same applies to the expressions of "2 h/2 h" and "3 h/3 h".

[Table 5]

| | Irradiation condition | Atmospheric condition | | Spore liquid | | | | | | | | |
| | | | | Cladosporium | | | Penicillium | | | Aspergillus niger | | |
| | | Temperature | Humidity | #6 No-load state | #1 Clean state | #2 Contaminated state | #6 No-load state | #1 Clean state | #2 Contaminated state | #6 No-load state | #1 Clean state | #2 Contaminated state |
|---|---|---|---|---|---|---|---|---|---|---|---|---|
| Comparative Example 3 | Not irradiated | 15-18°C | 30%RH | C | C | C | C | C | C | C | C | C |
| Comparative Example 4 | Not irradiated | 22-26°C | 40%RH | C | C | C | C | C | C | C | C | C |
| Example 2 | 222 nm 0.76 μW/cm² , 72 h | 15-18°C | 30%RH | A | A | A | A | A | A | A | A | A |
| Example 3 | 222 nm 0.29 μW/cm² , 72 h | 22-26°C | 40%RH | - | B1 | B1 | - | B1 | B1 | - | B1 | B1 |
| Example 4 | 222 nm 0.76 μW/cm² 45 min ON 15 min OFF, repeated for 72 h | 22-26°C | 40%RH | - | B1 | B1 | - | B1 | B1 | - | B1 | B1 |
| Example 5 | 222 nm 0.76 μW/cm² 30 min ON 30 min OFF, repeated for 72 h | 22-26°C | 40%RH | - | B2 | B2 | - | B2 | B2 | - | B2 | B2 |
| Example 6 | 222 nm 0.76 μW/cm2 2 h ON 2 h OFF, repeated for 72 h | 22-26°C | 40%RH | - | B2 | B2 | - | B2 | B2 | - | B2 | B2 |

(continued)

| | Irradiation condition | Atmospheric condition | | Spore liquid | | | | | | | | |
| | | | | Cladosporium | | | Penicillium | | | Aspergillus niger | | |
| | | Temperature | Humidity | #6 No-load state | #1 Clean state | #2 Contaminated state | #6 No-load state | #1 Clean state | #2 Contaminated state | #6 No-load state | #1 Clean state | #2 Contaminated state |
| Example 7 | 222 nm 0.76 μW/cm² 3 h ON 3 h OFF, repeated for 72 h | 22-26°C | 40%RH | - | B3 | B3 | - | B3 | B3 | - | B3 | B3 |
| Example 8 | 222 nm 0.76 μW/cm² 4 h ON 4 h OFF, repeated for 72 h | 22-26°C | 40%RH | - | B3 | B3 | - | B3 | B3 | - | B3 | B3 |

[Table 6]

| | Irradiation condition | Atmospheric condition | | Strain | | | | | | | | |
|---|---|---|---|---|---|---|---|---|---|---|---|---|
| | | | | Cladosporium | | | Penicillium | | | Aspergillus niger | | |
| | | Temperature | Humidity | #6 No-load state | #1 Clean state | #2 Contaminated state | #6 No-load state | #1 Clean state | #2 Contaminated state | #6 No-load state | #1 Clean state | #2 Contaminated state |
| Comparative Example 3 | Not irradiated | 15-18°C | 30%RH | C | C | C | C | C | C | C | C | C |
| Comparative Example 4 | Not irradiated | 22-26°C | 40%RH | C | C | C | C | C | C | C | C | C |
| Example 2 | 222 nm 0.76 $\mu$W/cm$^2$, 72 h | 22-26°C | 40%RH | A | A | A | A | A | A | A | A | A |
| Example 3 | 222 nm 0.29 $\mu$W/cm$^2$, 72 h | 15-18°C | 30%RH | B2 | B2 | B2 | B2 | B2 | B2 | B2 | B2 | B2 |
| Example 4 | 222 nm 0.76 $\mu$W/cm$^2$ 45 min ON 15 min OFF, repeated for 72 h | 22-26°C | 40%RH | B1 | B1 | B1 | B1 | B1 | B1 | B1 | B1 | B1 |
| Example 5 | 222 nm 0.76 $\mu$W/cm$^2$ 30 min ON 30 min OFF, repeated for 72 h | 22-26°C | 40%RH | B1 | B1 | B1 | B1 | B1 | B1 | B1 | B1 | B1 |
| Example 6 | 222 nm 0.76 $\mu$W/cm$^2$ 2 h ON 2 h OFF, repeated for 72 h | 22-26°C | 40%RH | B1 | B1 | B1 | B1 | B1 | B1 | B1 | B1 | B1 |

(continued)

| | Irradiation condition | Atmospheric condition | | Strain | | | | | | | | |
|---|---|---|---|---|---|---|---|---|---|---|---|---|
| | | | | Cladosporium | | | Penicillium | | | Aspergillus niger | | |
| | | Temperature | Humidity | #6 No-load state | #1 Clean state | #2 Contaminated state | #6 No-load state | #1 Clean state | #2 Contaminated state | #6 No-load state | #1 Clean state | #2 Contaminated state |
| Example 7 | 222 nm 0.76 $\mu$W/cm$^2$ 3 h ON 3 h OFF, repeated for 72 h | 22-26°C | 40%RH | B2 | B2 | B2 | B2 | B2 | B2 | B2 | B2 | B2 |
| Example 8 | 222 nm 0.76 $\mu$W/cm$^2$ 4 h ON 4 h OFF, repeated for 72 h | 22-26°C | 40%RH | B2 | B2 | B2 | B2 | B2 | B2 | B2 | B2 | B2 |

In Verification 2, the evaluation B used in Verification 1 is further divided into three levels of evaluation B1 to evaluation B3 in order to confirm the difference in the effect of the intermittent irradiation. That is, among samples in which a bacteriostatic effect was observed (evaluation B), samples in which the number of colonies was less than 30 are evaluated as B1, samples in which the number of colonies was 30 or more and less than 300 are evaluated as B2, and samples in which the number of colonies was 300 or more but hyphae were not observed are evaluated as B3. In addition, samples in which hyphae could be confirmed after a large number of colonies were formed are evaluated as C.

Note that a value less than 30 colonies corresponds to a detection limit or less microbiologically. In addition, a value not less than 300 colonies corresponds to a count limit.

According to Example 2, it was confirmed that the proliferation of the black mold could be suppressed even with ultraviolet light having an extremely low irradiance of 0.76 $\mu$W/cm$^2$ in both the case where the spore liquid was used and the strain was used. On the other hand, in Comparative Example 3, it was confirmed that colonies were formed in both the case where the spore liquid was used and the strain was used. In particular, according to Example 2, it was confirmed that the proliferation of the mold could be suppressed by irradiation with ultraviolet light having a wavelength of 222 nm even when the mold has already grown, as simulated by the samples #3 to #5.

According to Example 3, it was confirmed that the proliferation of the black mold could be suppressed even with ultraviolet light having an irradiance of 0.29 $\mu$W/cm$^2$ which was further lower than the irradiance in Example 2. It is to be noted, however, that the effect of suppressing the proliferation of the black mold is slightly reduced as compared with Example 2.

Examples 4 to 8 correspond to a case where ultraviolet light is intermittently emitted. It was confirmed that, in Examples 7 and 8 in which the non-irradiation time from the last irradiation was three hours or more, the effect of suppressing the proliferation of the black mold was reduced as compared with Examples 4 to 6 in which the non-irradiation time was two hours or less. That is, it is presumed that, even when the sample is irradiated with ultraviolet light for the same time (72 hours) with an average irradiance of the ultraviolet light radiated to the sample being similarly set to be equal to or less than $D_{Max}$, black molds tend to grow within the non-irradiation time as the non-irradiation time increases. In other words, it is found that the irradiation interval is preferably set within two hours when the ultraviolet light is intermittently radiated in order to suppress the proliferation of bacteria.

Comparing the photograph of the contaminated state (#4) of Comparative Example 4 shown in Fig. 9F and the photograph of Example 7 shown in Fig. 9J in which the conditions of the atmospheric temperature and the humidity were the same, hyphae can be observed in the photograph of Comparative Example 4, whereas hyphae cannot be observed in the photograph of Example 7, although it is difficult to recognize in the images of the photographs. From this result, it can be seen that the proliferation of bacteria was suppressed even in Example 7 in which ultraviolet light was radiated in a cycle of three hours, as compared with Comparative Example 3 in which ultraviolet light was not radiated. However, since the number of colonies is larger in Example 7 than in Example 4 and Example 6 shown in Fig. 9J, it is preferable to set the non-irradiation time to two hours or less from the viewpoint of further enhancing the bacteriostatic effect in the case of intermittently radiating ultraviolet light.

The verification was performed using ultraviolet light having an irradiance of 0.43 $\mu$W/cm$^2$ in Example 1, using ultraviolet light having an irradiance of 0.76 $\mu$W/cm$^2$ in Example 2, and using ultraviolet light having an irradiance of 0.29 $\mu$W/cm$^2$ in Example 3. In Examples 4 to 8 in which the samples were irradiated intermittently, the average irradiance was set to 0.57 $\mu$W/cm$^2$ in Example 4, and the average irradiance was set to 0.38 $\mu$W/cm$^2$ in Examples 5 to 8. However, it is obvious that the higher the irradiance, the higher the bacteriostatic effect can be. That is, by increasing the average irradiance within the range equal to or less than $D_{Max}$ ($\mu$W/cm$^2$) defined by above Formula (1), a high bacteriostatic effect can be exhibited with the influence on the human body being reduced.

[Another embodiment]

Another embodiment will be described below.

<1> In the above embodiment, the case of using the ultraviolet light irradiation device 1 equipped with the excimer lamps 3 as the light source has been described. However, the structure of the light source is not limited as long as the light source emits the ultraviolet light L1 having a main emission wavelength of 200 nm to 230 nm inclusive. For example, the light source may be a solid-state light source such as an LED or a semiconductor laser element.

In a case where the ultraviolet light irradiation device 1 includes a filter that blocks the light of 230 nm or more on the light extraction surface 10, a light source that also exhibits light output in a wavelength band of more than 230 nm can be used as long as the light output is exhibited in a part of the wavelength band of 200 nm to 230 nm. In this case, the main peak wavelength of the ultraviolet light L1 extracted from the light extraction surface 10 through the filter is 200 nm to 230 nm inclusive.

<2> To detect the irradiance of the ultraviolet light L1 emitted from the ultraviolet light irradiation device 1, an irradiance meter may be installed in a region irradiated with the ultraviolet light L1 (corresponding to the target region 40 in Fig. 1). As an example of such an irradiance meter, a spectral irradiance meter can be used, and specifically, USR-45D manufactured by USHIO INC. can be used.

The detection result of the irradiance meter may be transmitted to the ultraviolet light irradiation device 1. The ultraviolet light irradiation device 1 may include a control unit, and the control unit may calculate the average value of the irradiance by performing integration processing of the irradiance in a target wavelength band (200 nm to 230 nm). In addition, the irradiance meter may have an arithmetic processing function, and information regarding the average irradiance may be transmitted to the ultraviolet light irradiation device 1.

The control unit of the ultraviolet light irradiation device 1 may check whether or not the calculated average irradiance Ai is within a range equal to or less than $D_{Max}$ ($\mu$W/cm$^2$) defined by the above Formula (1). In this case, when Ai < $D_{Max}$, the control unit may control the light source mounted on the ultraviolet light irradiation device 1 so that the light source continuously emits the ultraviolet light L1, and when Ai approaches the value of $D_{Max}$ and Ai > $D_{Max}$ may be established soon, the control unit may reduce an output of the light source or turn off the light source so that Ai < $D_{Max}$ is continuously satisfied.

<3> In the above embodiment, any time may be set as the time used as the standard for calculating the "average irradiance", but as a typical example, the time may be the last eight hours in view of the ACGIH standard. That is, the average irradiance may be a value obtained by dividing the integral value of irradiance over the past eight hours based on the current time point by eight hours.

DESCRIPTION OF REFERENCE SIGNS

| | |
|---|---|
| 1 | Ultraviolet light irradiation device |
| 2 | Lamp house |
| 2a | Main body casing part |
| 2b | Lid part |
| 3 | Excimer lamp |
| 3G | Light-emitting gas |
| 8 | Feeder |
| 10 | Light extraction surface |
| 30 | Human sensor |
| 40 | Target region |
| 50 | Housing |
| $D_{Max}$ | Threshold irradiance |
| L1 | Ultraviolet light |

**Claims**

1. A bacteriostatic method for suppressing a proliferation of a bacterium in a target region, the method comprising a step (a) for irradiating the target region with ultraviolet light having a main peak wavelength of 200 nm to 230 nm inclusive at an average irradiance equal to or less than $D_{Max}$ ($\mu$W/cm$^2$) defined by Formula (1):

$$D_{Max} = 9391.1 \times \exp(-0.043\lambda) \bullet \bullet \bullet (1)$$

where $\lambda$ is the main peak wavelength (nm).

2. The bacteriostatic method according to claim 1, wherein the step (a) includes irradiating the target region with the ultraviolet light at an irradiance equal to or less than 1 $\mu$W/cm$^2$.

3. The bacteriostatic method according to claim 1 or 2, wherein

the bacterium is Staphylococcus aureus, and
the step (a) includes irradiating the target region with the ultraviolet light at an irradiance equal to or greater than 0.4 $\mu$W/cm$^2$.

4. The bacteriostatic method according to claim 1 or 2, wherein

the bacterium is a mold, and
the step (a) includes irradiating the target region with the ultraviolet light at an irradiance equal to or greater than 0.29 $\mu$W/cm$^2$.

5. The bacteriostatic method according to any one of claims 1 to 4, further comprising:

a step (b) for detecting whether or not a human is present in the target region by a human sensor; and
a step (c) for irradiating the target region with the ultraviolet light at an average irradiance higher than $D_{Max}$ ($\mu$W/cm$^2$) defined by the Formula (1), wherein,
when it is detected in the step (b) that a human is present in the target region, the method transitions from the step (c) to the step (a).

6. The bacteriostatic method according to any one of claims 1 to 5, wherein the step (a) includes radiating the ultraviolet light from an excimer lamp in which a light-emitting gas containing Kr and Cl is sealed.

**Fig. 1**

**Fig. 2**

Fig. 3

**Fig. 4**

**Fig. 5**

## Fig. 6

Graph legend:
- ◇— ACGIH-TLV Value (a)
- --◦-- Safety Factor 0.9 (b)
- ■— D_max (c)

## Fig. 7

**Fig. 8A**

Comparative Example 1 (Non-Irradiation)

| No-Load State | Clean State | Contaminated State |
|:---:|:---:|:---:|

**Fig. 8B**

Comparative Example 2 (254 nm, 0.22 µW/cm$^2$)

| No-Load State | Clean State | Contaminated State |
|---|---|---|

**Fig. 8C**

Example 1 (222 nm, 0.43 $\mu W/cm^2$)

| No-Load State | Clean State | Contaminated State |
|---|---|---|

**Fig. 9A**

Comparative Example 3
(Non-Irradiation: Spore Liquid of Cladosporium)

| #6 No-Load State | #1 Clean State | #2 Contaminated State |
|---|---|---|

**Fig. 9B**

Comparative Example 3
(Non-Irradiation: Strain of Cladosporium)

| #5 No-Load State | #3 Clean State | #4 Contaminated State |
| --- | --- | --- |

## Fig. 9C

Example 2: Spore Liquid of Cladosporium
(222 nm, 0.76 μW/cm$^2$, Continuous Irradiation)

| #6 No-Load State | #1 Clean State | #2 Contaminated State |
|---|---|---|

EP 4 109 493 A1

**Fig. 9D**

Example 2: Strain of Cladosporium
(222 nm, 0.76 µW/cm², Continuous Irradiation)

| #5 No-Load State | #3 Clean State | #4 Contaminated State |
|---|---|---|

**Fig. 9E**

Comparative Example 4: Spore Liquid of Cladosporium
(Non-Irradiation)

| #1 Clean State | #2 Contaminated State |
| --- | --- |

EP 4 109 493 A1

**Fig. 9F**

Comparative Example 4: Strain of Cladosporium
(Non-Irradiation)

| #3 Clean State | #4 Contaminated State |
|---|---|

EP 4 109 493 A1

**Fig. 9G**

Example 3: Spore Liquid of Cladosporium
(222 nm, 0.29 µW/cm², Continuous Irradiation)

#1 Clean State

#2 Contaminated State

**Fig. 9H**

Example 3: Strain of Cladosporium
(222 nm, 0.29 µW/cm$^2$, Continuous Irradiation)

| #3 Clean State | #4 Contaminated State |
|---|---|
| | |

Fig. 9I

Spore Liquid of Cladosporium
(222 nm, 0.76 µW/cm², Intermittent Irradiation)

Example 4 (45 minutes / 15 minutes) · Example 6 (2 hours / 2 hours) · Example 7 (3 hours / 3 hours)

**Fig. 9J**

Strain of Cladosporium
(222 nm, 0.76 μW/cm², Intermittent Irradiation)

| Example 4<br>(45 minutes / 15 minutes) | Example 6<br>(2 hours / 2 hours) | Example 7<br>(3 hours / 3 hours) |
| --- | --- | --- |

## INTERNATIONAL SEARCH REPORT

| | International application No. |
|---|---|
| | PCT/JP2021/005445 |

**A. CLASSIFICATION OF SUBJECT MATTER**
H01J 65/00(2006.01)i; A61L 2/10(2006.01)i; H01J 61/16(2006.01)i
FI: A61L2/10; H01J61/16 N; H01J65/00 A

According to International Patent Classification (IPC) or to both national classification and IPC

**B. FIELDS SEARCHED**

Minimum documentation searched (classification system followed by classification symbols)
H01J65/00; A61L2/10; H01J61/16

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 48-015517 Y1 (TOKYO SHIBAURA ELECTRIC CO., LTD.) 28 April 1973 (1973-04-28) column 1, line 26 to column 3, line 18, column 5, lines 5-8 | 1-6 |
| A | JP 2019-072411 A (USHIO INC.) 16 May 2019 (2019-05-16) claim 1, paragraphs [0001], [0007], [0014], [0038] | 1-6 |
| A | JP 2014-030763 A (EHIME UNIVERSITY) 20 February 2014 (2014-02-20) claim 1, paragraphs [0001], [0040] | 1-6 |
| A | JP 2014-136113 A (OBAYASHI CORPORATION) 28 July 2014 (2014-07-28) claim 1, paragraphs [0001], [0015] | 1-6 |
| A | JP 2018-517488 A (THE TRUSTEES OF COLUMBIA UNIVERSITY IN THE CITY OF NEW YORK) 05 July 2018 (2018-07-05) claims, paragraphs [0002], [0022], [0053] | 1-6 |

☐ Further documents are listed in the continuation of Box C.　　☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 20 April 2021 (20.04.2021) | 27 April 2021 (27.04.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

INTERNATIONAL SEARCH REPORT

Information on patent family members

International application no.

PCT/JP2021/005445

| Patent Documents referred in the Report | Publication Date | Patent Family | Publication Date |
|---|---|---|---|
| JP 48-015517 Y1 | 28 Apr. 1973 | (Family: none) | |
| JP 2019-072411 A | 16 May 2019 | JP 6497427 B1<br>WO 2019/077817 A1<br>TW 201922298 A | |
| JP 2014-030763 A | 20 Feb. 2014 | (Family: none) | |
| JP 2014-136113 A | 28 Jul. 2014 | (Family: none) | |
| JP 2018-517488 A | 05 Jul. 2018 | US 2018/0169279 A1<br>paragraphs [0002],<br>[0042], [0073],<br>claims 1-31<br>WO 2016/196904 A1<br>EP 3302328 A1<br>CN 107613895 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014136113 A